Europäisches Patentamt

European Patent Office

Office européen des brevets

(11) Publication number: **0 213 552**
A2

# (12) EUROPEAN PATENT APPLICATION

(21) Application number: **86111544.2**

(22) Date of filing: **20.08.86**

(51) Int. Cl.⁴: **A 61 K 9/10**
**A 61 K 47/00**

(30) Priority: **23.08.85 JP 186513/85**

(43) Date of publication of application:
**11.03.87 Bulletin 87/11**

(84) Designated Contracting States:
**AT BE CH DE FR GB IT LI LU NL SE**

(71) Applicant: Fujisawa Pharmaceutical Co., Ltd.
3, Doshomachi 4-chome Higashi-ku
Osaka-shi, Osaka 541(JP)

(72) Inventor: Ueda, Yoshio
No. 1-3-5-204, Mikagenaka-machi Higashinada-ku
Kobe-shi Hyogo(JP)

(72) Inventor: Kimura, Sumihisa
No. 2-13-1-408, Minamihibarigaoka
Takarazuka-shi Hyogo(JP)

(72) Inventor: Kohno, Yutaka
No. 3-7-407, Arujihara-cho
Ibaraki-shi Osaka(JP)

(74) Representative: Türk, Dietmar, Dr. rer. nat. et al,
Türk, Gille + Hrabal Patentanwälte Bruckner Strasse 20
D-4000 Düsseldorf 13(DE)

(54) Oily suspension for intrarectal infusion.

(57) Oily suspension for intrarectal infusion characterized in that water-soluble drug and gelling agent are suspended in oily substance by using dispersing agent and use thereof as a medicament.

EP 0 213 552 A2

# Oily suspension for intrarectal infusion

This invention relates to an oily suspension for intrarectal infusion in which water-soluble drug and gelling agent are suspended in oily substance by using dispersing agent and this invention is utilized in the therapeutical field.

Drugs which are poorly absorbed from the gastro-intestinal tract are usually administered by parenteral injection. However, parenteral injection has many disadvantages, for example, the problems of security and pain for the patients, the problem that the patients can not administer it by themselves, or the like, so the development of alternative dosage forms is greatly desired.

In this field, suppositories, which improve by adding the absorption-promoter the rectal absorption of the drugs as poorly absorbed from the gastro-intestinal tract, are eagerly studied.

Although said suppositories, which contain the absorption-promoter, improves bioavailability, they have many problems due to their preparation form.

Namely, the suppositories (i) are ruined in shape under relatively high temperature, (ii) contaminate hand(s) when administered to the patients, (iii) are felt like foreign substances in the rectum until they are dissolved, since they are solid form, (iv) can not be administered to the patient whose sphincter ani relaxes, etc.

The inventors of this invention have carried out extensive studies in order to overcome the above-mentioned problems and have found that suspension obtained by dispersing water-soluble drug and gelling agent into oily substance using dispersing agent had good bioavailability by the rectal administration.

Further, intrarectal infusion of the present invention is a new type intrarectal dosage form and has many advantages when compared with the above-mentioned suppositories.

Namely, intrarectal infusion of the present invention

(i) can be stored at ambient temperature,

(ii) can be administered rectally without contaminating hand(s)

(iii) can be administered in an optional dosage,

(iv) is not felt like a foreign substance in the rectum, since it is in a liquid form, etc.

The water-soluble drugs to be used in the present invention are usually the ones which are poorly absorbed from the gastro-intestinal tract.

Suitable water-soluble drug may be,

0213552

for example, Cephalospolin antibiotics (e.g. ceftizoxime, cefazolin, cephaloglycin, cephalothin, cephaloridine, their sodium salts etc., etc.), penicillin antibiotics (e.g. benzylpenicillin, carbenicillin, sulbenicillin, piperacillin, their sodium salts etc., etc.), amino glucoside antibiotics (e.g. gentamycin, streptomycin, kanamycin, paromomycin, fradiomycin, sisomicin, etc.), anti tumor substances (e.g. fluorouracil, mitomycin, adriamycin, bleomycin, etc.), peptide hormone (e.g. insulin, calcitonin, secretin, gastrin, somatomedin, etc.) or the like, but is not restricted to the above-mentioned drugs.

Gelling agents to be used in the present invention may be the ones which are set to gel by aqueous solution. Suitable gelling agent may be, for example, water-soluble polymer such as carboxyvinyl polymer or its salt [e.g. Carbopol (trademark; prepared by B.F. Goodrich Chemical Co., Ltd.) sodium salt], sodium polyacrylate, sodium alginate, sodium carboxymethylcellulose, carrageenan, xanthan gum, etc.
These gelling agents prevent the leak of drug and support the absorption of drug by being set to gel with aqueous solution after the administration of the intrarectal infusion of the present invention.
The dispersing agents to be used in the present invention may be the ones which can homogeneously disperse the water-soluble drug and the gelling agent in the oily substance.

Suitable dispersing agent may be, for example, surfactant such as glycerin mono fatty acid ester [e.g. MGS-B (brand name; prepared by Nikko Chemicals Co., Ltd.], dextrin fatty acid ester [e.g. Rheopearl KE (trademark; prepared by Kaihatsu Kagaku Co., Ltd.)],

sucrose fatty acid ester, sorbitan fatty acid ester, polyoxyethylene sorbitol fatty acid ester, polyoxyethylene fatty acid ester, polyoxyethylene higher alcohol ether, polyoxyethylene alkylaryl ether, propyleneglycol mono fatty acid ester, polyoxyethylene castor oil derivatives, polyoxypropylenepolyoxyethylenecetylalcohol ester or the like, alminum stearate, etc.

Among these dispersing agents, the surfactants which have low HLB value are preferable.

In case that the water-soluble drug is poorly absorbed from the gastro-intestinal tract, it is desirable to add absorption-promoter.

Suitable absorption-promoter may be the conventional one such as bile acid (e.g. cholic acid, glycocholic acid, taurocholic acid, deoxycholic acid, glycodeoxycholic acid, taurodeoxycholic acid, chenodeoxycholic acid, glycochenodeoxycholic acid, taurochenodeoxycholic acid, ursodeoxycholic acid, glycoursodeoxycholic acid, tauroursodeoxycholic acid, lithocholic acid, glycolithocholic acid, taurolithocholic acid, dehydrocholic acid, taurodehydrocholic acid, etc.) and their alkali metal salts, surfactant [e.g. HCO-60 (trademark; prepared by Nikko Chemicals Co., Ltd.), Span 60 (trademark; prepared by Atras Powder Co., Ltd.), etc.], fatty acids (e.g. sodium caprate, etc.), chelate (e.g. EDTA, etc.), alkaline earth metal salts of aldonic acid (e.g. calcium gluconate, etc.), salicylic acid, flufenamic acid or the like.

Among these absorption-promoters, cholic acid, glycocholic acid, taurocholic acid, chenodeoxycholic acid or glycochenodeoxycholic acid is more preferable due to weak rectal irritation and strong effect on absorption-enhancement.

Oily substance to be used in the present invention may be the one such as vegetable fatty oil (e.g. olive oil, soybean oil, sesami oil, safflower oil, rape seed oil, etc.), animal fatty oil (e.g. mink oil, egg oil, squalene, etc.), mono-, di- or tri-glyceride(s) of middle chain - saturated fatty acid [e.g. Coconad MT (trademark; prepared by Kao Food Co., Ltd.), Miglyol (trademark; prepared by Dynamit Novel Co., Ltd.), MGK (brand name; prepared by Nikko Chemicals Co., Ltd.), etc.], higher fatty acid (e.g. olelic acid, etc.), liquid lanolin, squalane or the like.

The ratio of water-soluble drug, gelling agent, dispersing agent and absorption-promoter in the oily suspension for intrarectal infusion of the present invention is usually 0.01-30 weight %, 0.1-10 weight %, 0.1-10 weight % and 0.1-10 weight % respectively, but is not restricted to the above ratio and is optionally determined. Preferable ratio of these components is shown in the below-mentioned Examples.

The oily suspension for intrarectal infusion of the present invention may contain conventional additives (e.g. antioxidants, antiseptics, etc.).

The oily suspension for intrarectal infusion of the present invention can be prepared by mixing and dispersing the above-mentioned components with the oily substance.

The oily suspension for intrarectal infusion thus obtained is poured into the container which is suitable for rectal administration and used.

To illustrate the effect of the present invention, test data on bioavailability are shown in the following.

## Bioavailability Test 1

### Test preparations

Sample A: Oily suspension for intrarectal infusion
disclosed in Example 1

Sample B: Suppository disclosed in Reference 1

### Test method

The aforesaid test preparations were administered rectally to four dogs for Sample A and to eight dogs for Sample B.

Dose of each test preparation was 250 mg potency as ceftizoxime sodium.

0.25, 0.5, 1, 2, 4 and 6 Hours after rectal administration, blood samples were collected and serum concentrations of ceftizoxime were measured by high-performance liquid chromatography.

AUC (Area under the serum concentration - time curve) from 0 to 6 hours after administration was calculated by trapezoidal method.

### Test result

Test result is shown in Table 1.

Table 1

|  | AUC 0-6 hr ($\mu g \cdot m\ell^{-1} \cdot hr$) |
|---|---|
| Sample A | 31.7 |
| Sample B | 30.8 |

The oily suspension for intrarectal infusion of the present invention has the same or superior bioavailability as compared with suppository.

Bioavailability Test 2

Test preparations

Sample C : Oily suspension for intrarectal infusion disclosed in Example 2

Sample D : Aqueous solution disclosed in Reference 2.

Test method

Test preparations were administered rectally to three rats.  Dose of each test preparation was 15 mg/kg as fluorouracil.

0.25, 0.5, 1, 1.5 and 2 Hours after the rectal administration, plasma concentrations of fluorouracil were measured and AUC from 0 to 2 hours after administration was calculated according to the same procedures respectively as described in Bioavailability Test 1.

Test result

Test result is shown in Table 2.

Table 2

| | $AUC_{0-2hr}$ ($\mu g \cdot m\ell^{-1} \cdot hr$) |
|---|---|
| Sample C | 3.55 |
| Sample D | 0.27 |

The oily suspension for intrarectal infusion of the present invention has much superior bioavailability to that of the aqueous solution.

## Bioavailability Test 3

### Test preparations

Sample E : Oily suspension for intrarectal infusion disclosed in Example 3.

Sample F : Saline suspension disclosed in Reference 3

### Test method

Test preparations were administered rectally to four dogs.

Dose of each test preparation was 5 international units/kg as insulin.

0.25, 0.5, 1, 2 and 3 Hours after rectal administration, plasma glucose levels were measured and change in plasma glucose was calculated.

### Test result

The result is shown in Table 3.

Table 3

| | Change in plasma glucose (%) | | | | |
| --- | --- | --- | --- | --- | --- |
| | 0.25 hr | 0.5 hr | 1 hr | 2 hr | 3 hr |
| Sample E | 43.4 | 41.5 | 41.1 | 17.3 | 6.6 |

Plasma glucose level was hardly decreased in case of administration of saline suspension in which dose was 10 international units/kg as insulin.

The oily suspension for intrarectal infusion of the present invention has much superior bioavailability to that of saline suspension.

The present invention is illustrated according to the examples as shown below, but is not limited thereto.

Example 1

|  |  |
|---|---|
| ceftizoxime sodium | 250 mg potency |
| Carbopol sodium | 62.5 mg |
| MGS-B | 75 mg |
| cholic acid | 112.5 mg |
| Coconad MT | suitable amount |
| total | 2500 mg |

Ceftizoxime sodium, Carbopol sodium, MGS-B and cholic acid were added to Coconad MT.

After the mixture was dispersed roughly by using homogenizer, the roughly dispersed mixture was dispersed by using Sonifier (trademark; prepared by Branson Sonic Power Co.) to give oily suspension for intrarectal infusion.

Example 2

|  |  |
|---|---|
| fluorouracil | 37.5 mg |
| Carbopol sodium | 30 mg |
| MGS-B | 30 mg |
| cholic acid | 48 mg |
| Coconad MT | suitable amount |
| total | 1200 mg |

Oily suspension for intrarectal infusion was obtained according to a similar procedure to that of Example 1.

Example 3

| | |
|---|---|
| insulin | 50 international units |
| Carbopol sodium | 62.5 mg |
| MGS-B | 75 mg |
| cholic acid | 112.5 mg |
| Coconad MT | suitable amount |
| total | 2500 mg |

Oily suspension for intrarectal infusion was obtained according to a similar procedure to that of Example 1.

Reference 1

| | |
|---|---|
| ceftizoxime sodium | 250 mg potency |
| sodium salt of capric acid | 50 mg |
| Witepsol H-5 | suitable amount |
| total | 1000 mg |

Witepsol H-5 (trademark; prepared by Dynamit Novel Co., Ltd.) was melted with heating and ceftizoxime sodium and sodium salt of capric acid as an absorption-promoter were added thereto and mixed.
The mixture was poured into the container for suppository.

Reference 2

The injection solution of fluorouracil (concentration: 50 mg/ml, prepared by Mitsui Seiyaku Co., Ltd.) was diluted with distilled water to give aqueous solution.

Reference 3

Insulin (38 mg, 26.5 international units/mg) was suspended in saline (10 ml) to give saline suspension.

What we claim is

1.  Oily suspension for intrarectal infusion characterized in that water-soluble drug and gelling agent are suspended in oily substance by using dispersing agent.

2.  Oily suspension for intrarectal infusion according to claim 1, wherein water-soluble drug is ceftizoxime sodium, fluorouracil or insulin.

3.  Oily suspension for intrarectal infusion according to claim 1, wherein gelling agent is carboxyvinyl polymer or its salt.

4.  Oily suspension for intrarectal infusion according to claim 1, wherein oily substance is glyceride of middle chain-saturated fatty acid.

5.  Oily suspension for intrarectal infusion according to claim 1, wherein dispersing agent is glycerin mono fatty acid ester.

6.  Use of an oily suspension of claim 1 as a medicament.